# EUROPEAN PATENT APPLICATION

(11) **EP 0 897 001 A1**
(43) Date of publication of application: **17.02.1999**
(21) Application number: 97114112.2
(22) Date of filing: 14.08.1997
(51) Int. Cl.: C12N 15/12, C07K 14/47, C07K 14/82, A61K 38/17, C12Q 1/68, C07K 16/32

(54) **Polypeptide having a GTPase regulator activity, nucleic acids coding therefore and their use in diagnostics and therapy**

(71) Applicant: Lampert, Fritz, Professor Dr., 35392 Giessen (DE); Borkhardt, Arndt, Dr., 35444 Biebertal (DE)
(72) Inventor: Lampert, Fritz, Professor Dr., 35392 Giessen (DE); Borkhardt, Arndt, Dr., 35444 Biebertal (DE)
(74) Representative: Keller, Günter, Dr.

(57) **Abstract**

The present invention discloses a polypeptide which is a GTPase regulator associated with focal adhesion kinase comprising the amino sequence

SHEAELDKTNKFIKELIKDGKSLISALKNLSSAKRKFADSLNEFKFQCIGDAETDDEMCIARS
LQEFATVLRNLEDERIRMIENASEVLITPLEKFRKEQIGAAKEAKKKYDKETEKYCGILEKHL
NLSSKKKESQLQEADSQVDLVRQHFYEVSLEYVFKVQEVQERKMFEFVEPLLAFLQGLFTFYH
HGYELAKDFGDFKTQLTISIQNTRNRFEGTRSEVESLMKKMKENPLEHKTISPYTMEGYLYVQ
EKRHFGTSWVKHYCTYQRDSKQITMVPFDQKSGGKGGEDESVILKSCTRRKTDSIEKRFCFDV
EAVDRPGVITMQALSEGDRRLWMEAMDGREPVYNSNKDSQSEGTAQLDSIGFSIIRKCIHAVE
TRGINEQGLYRIVGVNSRVQKLLSVLMDPKTASETETDICAEWEIKTITSALKTYLRMLPGPL
MMYQFQRSFIKAAKLENQESRVSEIHSLVHRLPEKNRQMLQLLMNHLANVANNHKQNLMTVAN
LGVVFGPTLLRPQEETVAAIMDIKFQNIVIEILIENHEKIFNTVPDMPLTNAQLHLSRKKSSD
SKPPSCSERPLTLFHTVQSTEKQEQRNSIINSSLESVSSNPNSILNSSSSLQPNMNSSDPDLA
VVKPTRPNSLPPNPSPTSPLSPSWPMFSAPSSPMPTSSTSSDSSPVSTPFRKAKALYACKAEH
DSELSFTAGTVFDNVHPSQEPGWLEGTLNGKTGLIPENYVEFL.

## Description

The present invention discloses polypeptides and polynucleotides derived from a human gene which has antiproliferative and tumor suppressor properties and the use thereof in diagnostics and therapy of cancer, in particular of myelodysplastic syndromes and acute myeloid leukemias.

Chromosome abnormalities associated with hematologic malignancies alter the normal structure and function of genes that control cell proliferation and differentiation either in a positive or negative fashion. Normal cell division is positively regulated or activated through signal transduction pathways composed of extracellular signals, receptor G proteins, protein kinases and transcription factors. The genes encoding such proteins are also known as proto-oncogenes, because mutations turn them into dominant oncogenes with altered properties. Thus, disrupture of one allele is generally sufficient to disturb normal cell division and initiate a neoplastic phenotype. Such proto-oncogenes are frequently affected by chromosome translocations. As a result, fusion of the participating genes leads to either abnormal activation or to the generation of novel chimeric oncogenes with new functions.

The majority of translocations that occur in chromosome 11 at 11(q23) in acute leukemias disrupt the mixed-lineage leukemia (*MLL*) gene within a small region of 8,6 kb and fuse it to a variety of different partner genes. The breakpoints within *MLL* are tightly clustered and therefore allow the detection of rearrangements on the DNA and RNA level in virtually all cases.

Chromosomal abnormalities associated with distinct subsets of human leukemia suggest a link between specific genetic alterations and clinical outcome. A tide correlation between the cytogenic anomaly and the progression of the disease indicates a direct cause and effect relationship between the genotype and the disease phenotype. Acquired interstitial loss of the long arm of chromosome 5 (5q-chromosome) is seen in patients with a range of myeloid disorders. A certain region in the long arm of chromosome 5 is consistently deleted in most of these patients suggesting that physical loss of an important gene from the 5q-chromosome coupled with a mutation of the second allele on the remaining "normal" chromosome 5 results in total inactivation of this key regulatory gene.

The different regions of the chromosome affected by the deletions can be designated by the commonly used nomenclature. The designation 5q31 for example means that the long arm (q) of chromosome 5 (5) is affected. The long arm of chromosome 5 is divided in three main areas (3; first digit) whereby said areas are further subdivided into five areas (second digit; here area 1).

The products of many so-called tumor suppressor genes inhibit the same signal transduction pathway and are negative regulators of the cell cycle. Since mutations in these genes act recessively and result in a loss of function, uncontrolled cell growth takes place only after inactivation or elimination of both alleles. Most often, one allele is deleted, whereas the second one may be functionally compromised by various other mutations. The most common structural aberrations encountered in myelodysplastic syndromes (MDS) and acute myeloid leukemias (AML) are deletions of the long arm of chromosome 5. Although the size and position of the deleted segments may vary considerably, band 5q31.1 is consistently lost in 90% of cases. This finding led to the notion that this critical region could harbor a tumor suppressor gene whose loss or inactivation is critical for the development of these malignant myeloid disorders. However, the inhomogeneity of the deletions, the paucity of highly polymorphic markers and the large number of attractive candidate genes within this region, has hampered the identification and isolation of such a gene considerably.

In the course of the present invention an infant with juvenile chronic myelomonocytic leukemia (JCMML) having an extremely rare chromosome translocation, namely t(5;11)(q31;q23) has been further investigated.

By using a unique t(5;11)(q31;q23) chromosome aberration in an infant with JCMML and a *MLL* gene rearrangement another *MLL* fusion partner gene could be cloned. In the course of the present invention a new member of the GTPase activating protein (GAP) family could be identified. Its position within the smallest region of genetic loss of 5q and its growth inhibitory and antioncogenic characteristics led us to investigate its possible involvement in hematologic neoplasms with 5q abnormalities. The fact that Southern-blot analysis of 20 such samples revealed rearrangements in 11 of them attributes this gene an important pathogenetic role in these diseases.

Integrin-mediated cell adhesion to the extracellular matrix (ECM) has both structural and biochemical ramifications for cell homeostasis. Integrins hind components of the ECM via large extracellular domains. The sites of integrin-ECM interaction trigger the formation of protein complexes on the cytoplasmic face of the plasma membrane termed focal adhesions. Focal adhesions contain several proteins with the capacity to simultaneously bind the short cytoplasmic tails of integrin and actin filaments. Thus, focal adhesions serve to anchor the actin cytoskeleton to the plasma membrane and directly link the extracellular and intracellular environments. Besides playing a structural role, the binding of integrins to ECM results in the initiation of intracellular signaling cascades. Activation of these cascades is believed to regulate gene expression, cell survival, cell migration and adhesion, and differentiation. A potential mediator of integrin signaling is the cytoplasmic protein kinases focal adhesion kinase (FAK). It goes without saying that a regulator of FAK is highly valuable in diagnosis and therapy. The polypeptide described in the present application is a GTPase regulator associated with FAK and in short termed "Graf".

The *Graf* gene encodes a regulator of the Rho family of small GTPases that binds to a tyrosine kinase. The Rho family belongs to the *RAS* superfamily and consists of five distinct types of GTP-binding proteins: RhoA, B & C; Rac1 & 2; Cdc42 & G25K; TC10 and RhoG, respectively. Rho, Rac and Cdc42 regulate the organization of the actin cytoskeleton. They control the assembly of actin stress fibers and focal adhesion complexes which anchor the actin cytoskeleton to the plasma membrane in normal cells, including neutrophils, lymphocytes, as well as platelets. The Graf protein of the present invention binds to the C-terminal domain of pp125^{FAK}, one of the tyrosine kinases predicted to be a critical component of the integrin signaling transduction pathway, in a SH3 domain-dependent manner and may thus preferentially stimulate the GTPase activity of the GTP-binding proteins RhoA and Cdc42. Amongst several other effects, this might initiate intracellular signaling cascades that participate in gene expression, cell survival, cell migration and adhesion, and differentiation. Moreover, Rho family GTPases also play a role in the regulation of growth control, because each of the three GTPases Rho, Rac and Cdc42 can induce Swiss 3T3 cells to progress through G1 and to enter the S phase of the cell cycle. Conversely, inhibitors of Rho, Rac and Cdc42 block serum induced DNA synthesis. The notion that Graf may have pleiotropic effects on integrin-mediated cell signaling is further corroborated by its presence in a variety of tissues, with a particular abundant expression in avian embryonic brain and liver and, as found in the course of the present invention, in human heart, brain and placenta.

Unlike *RAS*, the most frequently mutated proto-oncogene in human tumors, mutational activation of Rho related GTPases has not yet been detected in human malignancies. Nevertheless, it has been postulated that Rho and Cdc42 must have important roles in the development of human tumors, because many regulators of Rho have oncogenic activity. The mitigation or elimination of the negative regulatory function of the *Graf* might permanently activate Rho and Cdc42. Such a disturbance could significantly contribute to many neoplastic features, including factor and anchorage independent growth, invasion and metastasis. A biallelic loss or inactivation of *Graf* could thus be one of the molecular switches initiating neoplastic transformation in hematologic neoplasms with a del(5q).

The *Graf* gene is the second GAP-encoding gene involved in a leukemia-associated chromosomal translocation. The only other one currently known is the highly homologous *BCR* gene at chromosome 22q11 which is fused to the *ABL* gene in chronic myeloid and acute lymphoblastic leukemias harboring a t(9;22)(q34;q11). Interestingly, the functionally important GAP domain is lost in both the predicted hybrid proteins BCR/ABL and MLL/Graf. Whether the partial homology to the bombesin-like family members is functionally important remains open. Like cystokinin, bradykinin and substance P, the bombesins are bioactive peptides that were originally isolated from amphibian skin. Some of the bombesin-like peptides have been implicated in the pathogenesis of a number of mammalian carcinomas. Bombesin-like immunoreactivity (BLI) is found at high concentrations in fetal and neonatal lung, but nearly absent in adult lung tissue. In contrast, lung cancer cell lines and human tumors seem to contain much higher levels of BLI. Within this context it is noteworthy that *Graf* is also highly expressed in an adenocarcinoma cell line of the lung, but absent in normal lung tissue.

If *Graf* is actually one of the long searched tumor suppressor genes that are considered to participate in the neoplastic transformation process in myeloid disorders with del(5q), both alleles should be either absent or defective. The cytogenetic extensions of the deletions indicate that one allele of the *Graf* gene is lost in the majority of our rearranged cases. The normal bands comigrating with the abnormal ones most likely derive from residual normal cells rather than from an unaffected second allele in the neoplastic cells. The fact that rearrangements were not present in all our samples can be partly attributed to our unstrict selection criteria that also took into account cases with rather complex karyotypes and even some that did not affect 5q31 on the cytogenetic level. Finally, our probe only covered the 3'-part of the Graf gene and, given the rather heterogeneous extensions of 5q deletions, several other pathogenetically relevant genes might exist in this particular region.

Since the Graf gene and the polypeptide encoded by said gene are of significant importance in therapy and diagnosis, the present invention concerns a polypeptide having the amino acid sequence 
SHEAELDKTNKFIKELIKDGKSLISALKNLSSAKRKFADSLNEFKFQCIGDAETDDEMCIARS
LQEFATVLRNLEDERIRMIENASEVLITPLEKFRKEQIGAAKEAKKKYDKETEKYCGILEKHL
NLSSKKKESQLQEADSQVDLVRQHFYEVSLEYVFKVQEVQERKMFEFVEPLLAFLQGLFTFYH
HGYELAKDFGDFKTQLTISIQNTRNRFEGTRSEVESLMKKMKENPLEHKTISPYTMEGYLYVQ
EKRHFGTSWVKHYCTYQRDSKQITMVPFDQKSGGKGGEDESVILKSCTRRKTDSIEKRFCFDV
EAVDRPGVITMQALSEGDRRLWMEAMDGREPVYNSNKDSQSEGTAQLDSIGFSIIRKCIHAVE
TRGINEQGLYRIVGVNSRVQKLLSVLMDPKTASETETDICAEWEIKTITSALKTYLRMLPGPL
MMYQFQRSFIKAAKLENQESRVSEIHSLVHRLPEKNRQMLQLLMNHLANVANNHKQNLMTVAN
LGVVFGPTLLRPQEETVAAIMDIKFQNIVIEILIENHEKIFNTVPDMPLTNAQLHLSRKKSSD
SKPPSCSERPLTLFHTVQSTEKQEQRNSIINSSLESVSSNPNSILNSSSSLQPNMNSSDPDLA
VVKPTRPNSLPPNPSPTSPLSPSWPMFSAPSSPMPTSSTSSDSSPVSTPFRKAKALYACKAEH
DSELSFTAGTVFDNVHPSQEPGWLEGTLNGKTGLIPENYVEFL (Seq.-ID Nr. 1)
 or a fragment thereof with at least 70 amino acids.

Since the polypeptides of the present invention are derived from a human gene it should be noted that also such derivatives are biologically active which differs somewhat from the amino acid sequence as given above. The present invention comprises therefore also such fragments of the polypeptides which have at least a partial sequence derived from the disclosed amino sequence whereby said partial fragment comprises preferably more than 85 and more preferably more than 100 consecutive amino acids of the above given sequence. The underlined regions are especially preferred.

The polypeptide of the present invention, which is called "Graf", is a GTPase regulator associated with focal adhesion kinase and most likely a GTPase-activating protein.

The polypeptide of the present invention can be used in a pharmaceutical composition comprising such a polypeptide.

The pharmaceutical composition of the present invention can be used for the treatment of cancer, whereby the pharmaceutical composition is preferably formulated for parenteral application.

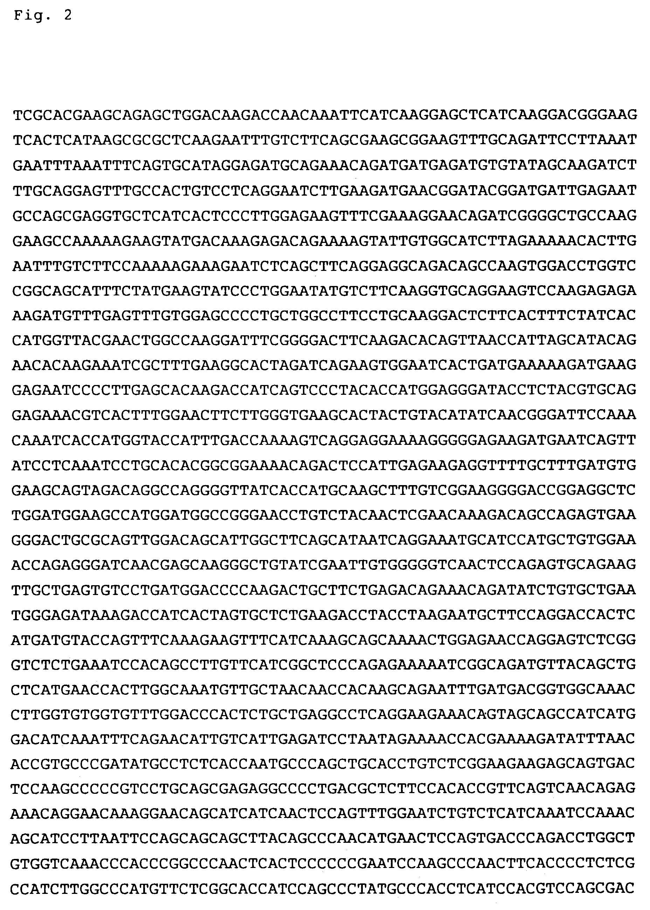

Another aspect of the present invention concerns polynucleotides coding for said Graf gene comprising the nucleotide sequence as shown in Figure 2 (Seq.ID-Nr. 2) or a partial sequence thereof having at least 15 consecutive nucleotides.

A partial sequence of the nucleotide sequence given above has preferably at least 25 consecutive nucleotides and more preferably at least 50 consecutive nucleotides.

The polynucleotides of the present invention comprise preferably also sequences which are required in order to allow the polynucleotide to replicate in a host cell. Those sequences are well-known in the art and are essential for a vector in order to replicate in a host cell. The vectors of the present invention are preferably a plasmid or a viral vector comprising a polynucleotide of the present invention. Such a vector is used in order to transform a suitable host cell in order to cause said host cell to express a polypeptide according to the present invention.

Another aspect of the present invention concerns testkits for the diagnosis of chromosomal aberrations by hybridization of nucleic acids. Said testkits comprise at least a polynucleotide of the present invention. Those testkits are preferably testkits for performing a Southern Blot hybridization or for performing a polymerase chain reaction. Beside the polynucleotides of the present invention such kits comprise frequently used components which are well-known to the person skilled in the art.

In another aspect of the present invention a polypeptide can be used for the preparation of antibodies. The technique of producing antibodies or monoclonal antibodies is well-known to an average person skilled in the art.

The present invention is further illustrated by the following examples.

### Example 1

### Isolation of the human Graf gene and its expression in human tissues

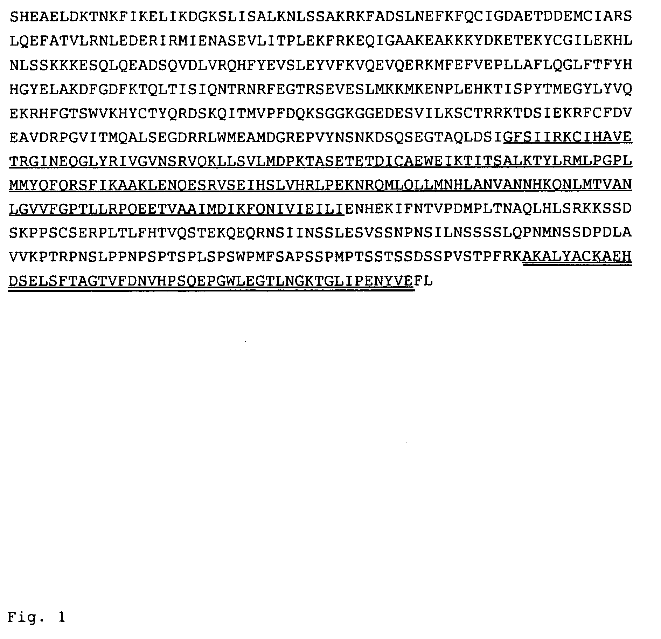

Cytogenetic analysis of a bone marrow sample obtained from a child with JCMML revealed a unique translocation t(5;11)(q31;q23). Southern-blot analysis and fluorescence in situ hybridization (FISH) subsequently confirmed that this translocation disrupted the MLL gene. This enabled the isolation of the fusion partner with a 5' and 3' rapid amplification of cDNA ends polymerase chain reaction (RACE RT-PCR) technique. A 3089 nucleotide long consensus sequence with an open reading frame of 736 amino acids was recovered. A search of the PROSITE database revealed a guanosine triphosphatase activating (GAP) domain (amino acids 366 to 539) and an SH3 domain (amino acids 683 to 734) at the carboxy-terminus of the protein (Figure 1). Homology scans indicated that this is a new GTPase activating protein for the Rho family, with 90 % protein homology to the product of a recently isolated cDNA clone obtained from a chicken library. Because the SH3 domain of this particular avian protein mediates binding to the pp125 focal adhesion kinase (pp125^{FAK}) protein, this clone was termed *Graf* (for GTPase regulator associated with pp125^{FAK}). Analysis of the human *Graf* protein sequence with the MOTIF software revealed domains of the immunoglobulin and major histocompatibility complex proteins at amino acids 687-693 and a bipartite nuclear targeting sequence (amino acids 97 to 113), whereas analysis with the BLIMPS package programme (EMBL, Heidelberg, Germany) detected two different partial homologies with the bombesin-like family of proteins at amino acids 554-564 and 715-725, respectively. Northern-blot analysis of the human *Graf* disclosed a major and a minor transcript with 9,5 and 4,4 kb, respectively. A Zoo blot which was performed with a 1167 bp clone showed that this gene is conserved in all the examined mammalian tissues including monkey, cow and dog. The probe did not hybridize to yeast DNA.

### Example 2

### Molecular analysis of the t(5;11)(q31;q23)

In *MLL*, the break occurred after exon 7 and in the *Graf* gene at nucleotide numbers 1629/30. Exon 6 of the *MLL* gene is deleted in the *MLL/Graf* hybrid mRNA. However, the maintenance of the open reading frame would enable the formation of a new chimeric *MLL/Graf* protein. The predicted fusion protein consists of the N-terminal part of *MLL* and the C-terminal part of *Graf*. It retains the putative "AT-hook" DNA binding domain and the DNA methyltransferase motifs present in the *MLL* amino-terminal region, and the SH3 domain of the *Graf* gene, whereas the GAP domain of *Graf* is deleted.

RT-PCR revealed that the fusion mRNA of the *MLL/Graf* but not that of the reciprocal *Graf/MLL* is expressed. With long-range PCR, an approximately 13 kb fragment of the *MLL/Graf* sequence on the genomic DNA level in the case with the t(5;11), but in none of four leukemic cell lines and 3 healthy individuals could be amplified. Using the primer pairs G1/G3 and G2/G4 that are positioned at nucleotide numbers 1559-1591 (G1), 1736-1608 (G3), 1593-1625 (G2) and 1674-1645 (G4), respectively, we amplified an approximately 11 kb long intron of the *Graf* gene in which the break and fusion with *MLL* had taken place.

### Example 3

### Chromosomal assignment of the Graf gene to 5(q31)

Hybridization of a 1167 bp cDNA clone of the human *Graf* gene onto normal metaphase chromosomes confirmed its position at 5(q31).

### Example 4

### Rearrangements of the human Graf gene in patients with MDS/AML and 5q aberrations

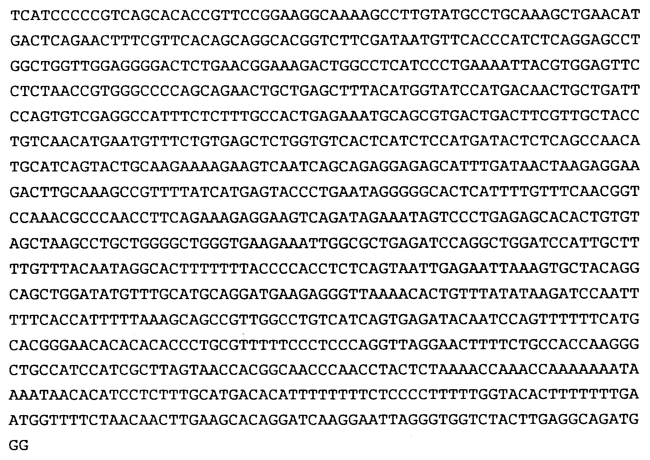

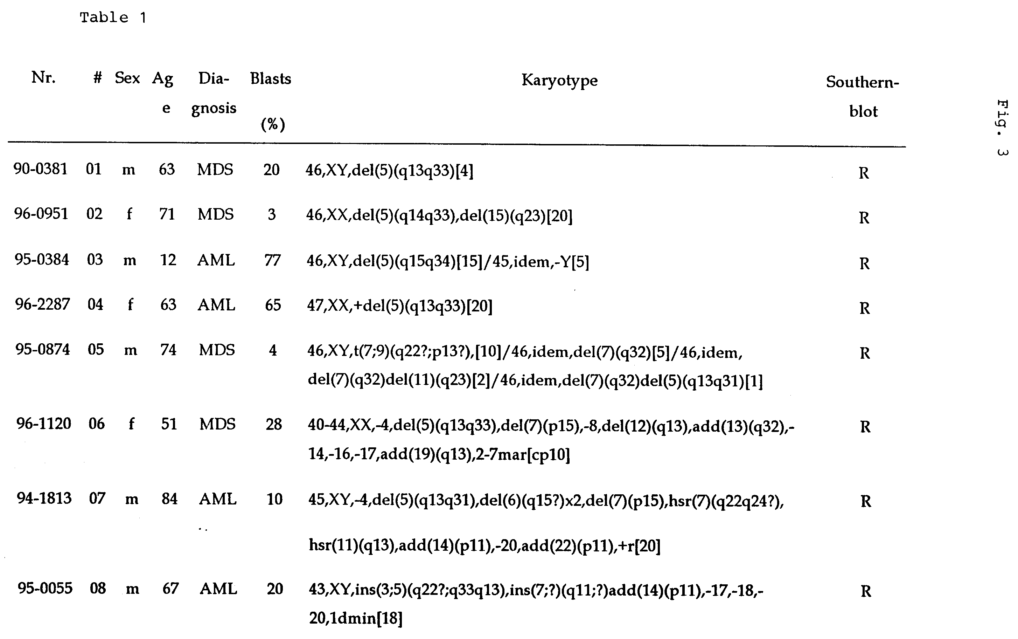

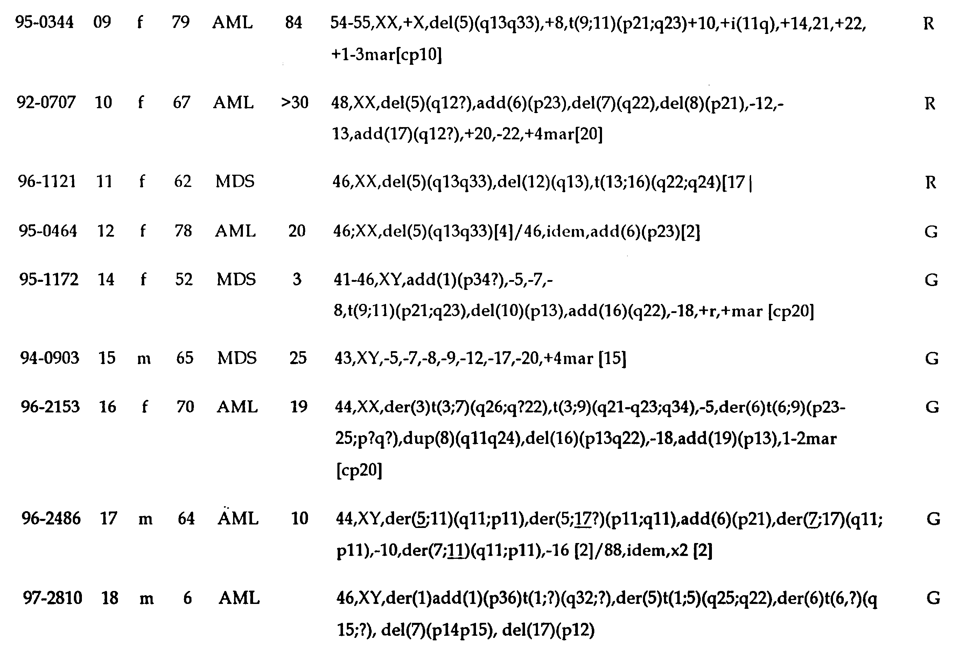

To assess a possible involvement of the *Graf* gene in hematologic neoplasms we performed Southern-blot analyses on samples of 20 patients with abnormalities of 5q. The results of this experiment are summarized in Table 1 as shown in Figure 3.

Three patients (#1-3) had a pure interstitial deletion and another one (#4) an additional del(5) as the only karyotype abnormality. Nine patients (#5-13) had a complex karyotype that contained an interstitial del(5q). However, the complexity of the changes and the presence of unidentifiable marker chromosomes prevented the unequivocal exclusion of an underlying balanced rearrangement with cytogenetic means. This was also the case in two patients with a cytogenetically apparent loss of chromosome 5 (# 14 & 15). In the remaining five cases (#16-20) the complex changes involved regions other than 5(q31).

In 11 of the 20 neoplastic samples analyzed, we found additional bands. Thus, a rearrangement of the *Graf* gene was present in all four patients with a pure interstitial deletion of 5(q) and in seven of nine patients in whom this abnormalitiy was part of a complex karyotype. However, with the applied probe and enzyme we were unable to detect any rearrangement in the other patients with complex karyotypes and either a monosomy 5 or affecting breakpoints other than 5(q31).

To exclude the possibilities that these rearrangements were due to DNA polymorphisms within the investigated region of the *Graf* gene, we analyzed ten healthy individuals, three permanent leukemic cell lines without 5q deletions (RS411, MV411 and K562) and ten samples from leukemias with various other MLL rearrangements. All these controls revealed an identical normal germline pattern.

### Example 5

### Southern-, Northern-, multiple tissue- and Zoo-blot.

High molecular weight DNA was isolated according to standard pocedures. Aliquots of 5 µg DNA were digested with Hind III restriction endonuclease (Boehringer Mannheim, Germany). The DNA was separated by electrophoresis in a 0,7 % agarose gel at 25 V over night and hybridized onto nylon membranes. The blots were probed with a cDNA clone of the human *Graf* gene ranging from nucleotide 225 to 1392. The cDNA probe was labelled by incorporating digoxigenine 11-dUTP. Filters were hybridized at 37°C for 10 h using the EasyHyb solution (Boehringer Mannheim, Germany), followed by two washes with 2xSSC/0,1 % SDS and 0,1xSSC/0,1 % SDS, respectively. The detection was carried out with an anti-digoxigenin antibody (Boehringer Mannheim, Germany) that was conjugated with alkaline phosphatase. The prefabricated Northern blots and the Zoo-blot were purchased from Clontech (Palo Alto, CA). The blots were hybridized according to the manufactures instructions with the same cDNA probe of the human *Graf* gene. For analysis of the MLL gene, the 859 bp-BamHI fragment of cDNA that spans the MLL-breakpoint cluster region defined by exons 5-11 of the MLL gene was used.

## Claims

1. Polypeptide comprising the amino sequence 
SHEAELDKTNKFIKELIKDGKSLISALKNLSSAKRKFADSLNEFKFQCIGDAETDDEMCIARS
LQEFATVLRNLEDERIRMIENASEVLITPLEKFRKEQIGAAKEAKKKYDKETEKYCGILEKHL
NLSSKKKESQLQEADSQVDLVRQHFYEVSLEYVFKVQEVQERKMFEFVEPLLAFLQGLFTFYH
HGYELAKDFGDFKTQLTISIQNTRNRFEGTRSEVESLMKKMKENPLEHKTISPYTMEGYLYVQ
EKRHFGTSWVKHYCTYQRDSKQITMVPFDQKSGGKGGEDESVILKSCTRRKTDSIEKRFCFDV
EAVDRPGVITMQALSEGDRRLWMEAMDGREPVYNSNKDSQSEGTAQLDSIGFSIIRKCIHAVE
TRGINEQGLYRIVGVNSRVQKLLSVLMDPKTASETETDICAEWEIKTITSALKTYLRMLPGPL
MMYQFQRSFIKAAKLENQESRVSEIHSLVHRLPEKNRQMLQLLMNHLANVANNHKQNLMTVAN
LGVVFGPTLLRPQEETVAAIMDIKFQNIVIEILIENHEKIFNTVPDMPLTNAQLHLSRKKSSD
SKPPSCSERPLTLFHTVQSTEKQEQRNSIINSSLESVSSNPNSILNSSSSLQPNMNSSDPDLA
VVKPTRPNSLPPNPSPTSPLSPSWPMFSAPSSPMPTSSTSSDSSPVSTPFRKAKALYACKAEH
DSELSFTAGTVFDNVHPSQEPGWLEGTLNGKTGLIPENYVEFL
 or a fragment thereof with at least 70 amino acids.

2. Polypeptide according to claim 1 characterized in that the fragment comprises at least 85 consecutive amino acids as given in the sequence of claim 1.

3. Polypeptide according to claim 1 characterized in that the fragment comprises at least 100 consecutive amino acids as given in the sequence of claim 1.

4. Polypeptide according to any one of claims 1 to 3 characterized in that it is a GTPase regulator associated with focal adhesion kinase.

5. Polypeptide according to claim 4 characterized in that the GTPase regulator is a GTPase-activating protein.

6. Pharmaceutical composition comprising a polypeptide according to any one of claims 1 to 5.

7. Pharmaceutical composition according to claim 6 for the treatment of cancer.

8. Pharmaceutical composition according to claims 6 or 7 for parenteral application.

9. Polynucleotide characterized in that it comprises the sequence 
TCGCACGAAGCAGAGCTGGACAAGACCAACAAATTCATCAAGGAGCTCATCAAGGACGGGAAG
TCACTCATAAGCGCGCTCAAGAATTTGTCTTCAGCGAAGCGGAAGTTTGCAGATTCCTTAAAT
GAATTTAAATTTCAGTGCATAGGAGATGCAGAAACAGATGATGAGATGTGTATAGCAAGATCT
TTGCAGGAGTTTGCCACTGTCCTCAGGAATCTTGAAGATGAACGGATACGGATGATTGAGAAT
GCCAGCGAGGTGCTCATCACTCCCTTGGAGAAGTTTCGAAAGGAACAGATCGGGGCTGCCAAG
GAAGCCAAAAAGAAGTATGACAAAGAGACAGAAAAGTATTGTGGCATCTTAGAAAAACACTTG
AATTTGTCTTCCAAAAAGAAAGAATCTCAGCTTCAGGAGGCAGACAGCCAAGTGGACCTGGTC
CGGCAGCATTTCTATGAAGTATCCCTGGAATATGTCTTCAAGGTGCAGGAAGTCCAAGAGAGA
AAGATGTTTGAGTTTGTGGAGCCCCTGCTGGCCTTCCTGCAAGGACTCTTCACTTTCTATCAC
CATGGTTACGAACTGGCCAAGGATTTCGGGGACTTCAAGACACAGTTAACCATTAGCATACAG
AACACAAGAAATCGCTTTGAAGGCACTAGATCAGAAGTGGAATCACTGATGAAAAAGATGAAG
GAGAATCCCCTTGAGCACAAGACCATCAGTCCCTACACCATGGAGGATACCTCTACGTGCAG
GAGAAACGTCACTTTGGAACTTCTTGGGTGAAGCACTACTGTACATATCAACGGATTCCAAA
CAAATCACCATGGTACCATTTGACCAAAAGTCAGGAGGAAAAGGGGGAGAAGATGAATCAGTT
ATCCTCAAATCCTGCACACGGCGGAAAACAGACTCCATTGAGAAGAGGTTTTGCTTTGATGTG
GAAGCAGTAGACAGGCCAGGGGTTATCACCATGCAAGCTTTGTCGGAAGGGGACCGGAGGCTC
TGGATGGAAGCCATGGATGGCCGGGAACCTGTCTACAACTCGAACAAAGACAGCCAGAGTGAA
GGGACTGCGCAGTTGGACAGCATTGGCTTCAGCATAATCAGGAAATGCATCCATGCTGTGGAA
ACCAGAGGGATCAACGAGCAAGGGCTGTATCGAATTGTGGGGGTCAACTCCAGAGTGCAGAAG
TTGCTGAGTGTCCTGATGGACCCCAAGACTGCTTCTGAGACAGAAACAGATATCTGTGCTGAA
TGGGAGATAAAGACCATCACTAGTGCTCTGAAGACCTACCTAAGAATGCTTCCAGGACCACTC
ATGATGTACCAGTTTCAAAGAAGTTTCATCAAAGCAGCAAAACTGGAGAACCAGGAGTCTCGG
GTCTCTGAAATCCACAGCCTTGTTCATCGGCTCCCAGAGAAAAATCGGCAGATGTTACAGCTG
CTCATGAACCACTTGGCAAATGTTGCTAACAACCACAAGCAGAATTTGATGACGGTGGCAAAC
CTTGGTGTGGTGTTTGGACCCACTCTGCTGAGGCCTCAGGAAGAAACAGTAGCAGCCATCATG
 
GACATCAAATTTCAGAACATTGTCATTGAGATCCTAATAGAAAACCACGAAAGATATTTAAC
ACCGTGCCCGATATGCCTCTCACCAATGCCCAGCTGCACCTGTCTCGGAAGAAGCAGTGAC
TCCAAGCCCCCGTCCTGCAGCGAGAGGCCCCTGACGCTCTTCCACACCGTTCAGTCAACAGAG
AAACAGGAACAAAGGAACAGCATCATCAACTCCAGTTTGGAATCTGTCTCATCAAATCCAAAC
AGCATCCTTAATTCCAGCAGCAGCTTACAGCCCAACATGAACTCCAGTGACCCAGACCTGGCT
GTGGTCAAACCCACCCGGCCCAACTCACTCCCCCCGAATCCAAGCCCAACTTCACCCCTCTCG
CCATCTTGGCCCATGTTCTCGGCACCATCCAGCCCTATGCCCACCTCATCCACGTCCAGCGAC
TCATCCCCCGTCAGCACACCGTTCCGGAAGGCAAAAGCCTTGTATGCCTGCAAAGCTGAACAT
GACTCAGAACTTTCGTTCACAGCAGGCACGGTCTTCGATAATGTTCACCCATCTCAGGAGCCT
GGCTGGTTGGAGGGGACTCTGAACGGAAAGACTGGCCTCATCCCTGAAAATTACGTGGAGTTC
CTCTAACCGTGGGCCCCAGCAGAACTGCTGAGCTTTACATGGTATCCATGACAACTGCTGATT
CCAGTGTCGAGGCCATTTCTCTTTGCCACTGAGAAATGCAGCGTGACTGACTTCGTTGCTACC
TGTCAACATGAATGTTTCTGTGAGCTCTGGTGTCACTCATCTCCATGATACTCTCAGCCAACA
TGCATCAGTACTGCAAGAAAAGAAGTCAATCAGCAGAGGAGAGCATTTGATAACTAAGAGGAA
GACTTGCAAAGCCGTTTTATCATGAGTACCCTGAATAGGGGGCACTCATTTTGTTTCAACGGT
CCAAACGCCCAACCTTCAGAAAGAGGAAGTCAGATAGAAATAGTCCCTGAGAGCACACTGTGT
AGCTAAGCCTGCTGGGGCTGGGTGAAGAAATTGGCGCTGAGATCCAGGCTGGATCCATTGCTT
TTGTTTACAATAGGCACTTTTTTTACCCCACCTCTCAGTAATTGAGAATTAAAGTGCTACAGG
CAGCTGGATATGTTTGCATGCAGGATGAAGAGGGTTAAAACACTGTTTATATAAGATCCAATT
TTTCACCATTTTTAAAGCAGCCGTTGGCCTGTCATCAGTGAGATACAATCCAGTTTTTTCATG
CACGGGAACACACACACCCTGCGTTTTTCCCTCCCAGGTTAGGAACTTTTCTGCCACCAAGGG
CTGCCATCCATCGCTTAGTAACCACGGCAACCCAACCTACTCTAAAACCAAACCAAAAAAATA
AAATAACACATCCTCTTTGCATGACACATTTTTTTCTCCCCTTTTTGGTACACTTTTTTTGA
ATGGTTTTCTAACAACTTGAAGCACAGGATCAAGGAATTAGGGTGGTCTACTTGAGGCAGATG
GG
 or a partial sequence thereof having at least 15 consecutive nucleotides.

10. Polynucleotide according to claim 9 characterized in that the partial sequence of the nucleotide sequence given in claim 9 has at least 25 consecutive nucleotides.

11. Polynucleotide according to claim 9 characterized in that the partial sequence of the nucleotide sequence given in claim 9 has at least 50 consecutive nucleotides.

12. Polynucleotide according to any one of claims 9 to 11 characterized in that it comprises also sequences which are required in order to allow the polynucleotide to replicate in a host cell.

13. Polynucleotide according to claim 12 characterized in that it is a vector suitable to replicate in a host cell.

14. Polynucleotide according to claim 13 characterized in that the vector a plasmid or a vector derived from a virus.

15. Host cell characterized in that said host cell is transformed with a polynucleotide according to claims 13 or 14 causing said host cell to express a polypeptide according to any one of claims 1 to 5.

16. Testkit for the diagnosis of chromosomal aberrations by hybridization of nucleic acids characterized in that said testkit comprises a polynucleotide according to any one of claims 9 to 14.

17. Testkit according to claim 16 characterized in that it is a testkit for performing a Southern Blot hybridization.

18. Testkit according to claim 16, characterized in that said testkit is a kit for performing a polymerase chain reaction.

19. Use of a polypeptide according to any one of claims 1 to 5 for the preparation of antibodies.
